# EUROPEAN PATENT APPLICATION

(11) **EP 2 314 251 A1**
(43) Date of publication of application: **27.04.2011**
(21) Application number: 09173565.4
(22) Date of filing: 20.10.2009
(51) Int. Cl.: A61C 8/00, A61L 27/06

(54) **Dental implant with nanotube surface with micrometric cavities**

(71) Applicant: Vrespa, Giuseppe, 20025 Legnano (MI) (IT)
(72) Inventor: Vrespa, Giuseppe, 20025, Legnano - Mi (IT); Bestetti, Massimiliano, 20133, Milano (IT)
(74) Representative: Ripamonti, Enrico

(57) **Abstract**

In the dental implant, of titanium or its alloys, at least that surface of the implant intended to come into contact with the gingival epithelium has a structure of the titanium oxide nanotube type, with cavities of micrometric dimensions.

## Description

The present invention relates to dental implants.

Implantology currently has a considerable remaining problem, i.e. the poor adhesion of the connective gingival tissue to the implant. This congenital weakness of the gingival epithelium/implant joint can be a source of infective attacks which are repeated with time and, according to statistics obtained from the literature on the subject, involve about 9.6% of the inserted implants. The poor resistance of this joint to infections results in the penetration of toxic substances of bacterial origin between the implant and the epithelium, giving rise to a series of infective problems which sometimes compromise the life of the implant itself.

It has been known for many years to improve the osteoconductive and osteoinductive properties of the surface of a titanium or titanium alloy implant, in order to improve adhesion of the bone tissue to the implant, by using anodic oxidation of the surface of such implants (in this respect see WO-A-2006104644).

Anodic oxidation is a technique which enables the oxide of a metal (typically aluminium or titanium) to artificially grow electrochemically. The metal piece to be oxidized is immersed in suitable solution and connected to the positive pole of an electric current generator, while an auxiliary electrode, also immersed in the solution, is connected to the opposite pole. The current circulation causes oxide growth.

The composition and temperature of the anodizing solution, the operating voltage and the duration of oxidation determine the anodic oxide characteristics. For further details reference should be made to the classic texts on the subject, for example E. Bertorelle, Trattato di galvanotecnica, Hoepli 1977; A. W. Brace, P.G. Sheasby, The technology of anodizing aluminium, Technicopy 1979; V.F. Henley, Anodic oxidation of aluminium and its alloys, Pergamon Press 1982.

In particular, the anodic oxidation of titanium enables anodic oxides of nanotube structure to be obtained on the metal surface. This was initially achieved using sulphuric and hydrofluoric acid based solutions (first generation solutions). A typical formulation for the aqueous solution is the following: 1M sulphuric acid and 0.15% hydrofluoric acid. The anodic oxidation is carried out at a cell voltage of 20 V at ambient temperature. The oxide nanotubes develop perpendicular to the titanium surface to give rise to nanotubes of maximum length 0.5-1,0 micrometres after 24 hours of treatment. The internal diameter of the nanotubes is between 50 and 200 nanometres (M. Bestetti et al., Structure of nanotubular titanium oxide templates prepared by electrochemical anodization in H2SO4/HF solutions, Thin Solid Films 2007, vol. 515, no. 13, pp. 5253-5258).

It has however been found that the nanotube structure on the surface of the dental implants, with regard to obtaining a good joint between the bone tissue and the implant in terms of the joint with the gingival epithelium, does not ensure sufficient resistance to infective attacks, not being optimized to obtain anchoring either with type IV collagen and with tropocollagen or with the tissue cell component.

The object of the present invention is to overcome the aforespecified drawback, in particular by providing a dental implant of titanium or its alloys by which the resistance to infective attacks of the joint between the gingival epithelium and the implant is substantially improved.

Before illustrating how the aforesaid object is attained, it should be noted that anodic oxidation techniques are known using viscous solvents in the absence of water (third generation solutions). The solutions mostly used are based on glycerol or dimethylformamide and fluorinated compounds. The anodization is carried out at a temperature between 0ºC and 80ºC at 20 V. The experimental data have shown that the diameter and length of the nanotubes strongly depend on the solution viscosity. The special characteristic of these anodic oxidation techniques is that they enable a surface to be obtained which is still of nanotube structure, but also presents cavities of micrometric dimensions with random distribution (Chuanmin Ruan et al., Fabrication of Highly Ordered TiO2 Nanotube Arrays Using an Organic Electrolyte, Journal of Physical Chemistry B, 2005, Vol.109, No. 33, pp 15754-15759).

Returning to the aforesaid object, this is attained by the dental implant of the present invention, consisting essentially of titanium or its alloys, in which at least that surface of the implant intended to come into contact with the gingival epithelium has a structure of the titanium oxide nanotube type, with cavities of micrometric dimensions.

An implant surface structured in this manner - at two levels: nano (the nanotubes) and micro (the cavities) - presents the advantage that while the microfibrils of the fibroblast cells (gingival fibroblasts) are housed in the nanotubes, both entire fibroblast cells and cytoplasmic prolongations of these cells are housed in the micrometric cavities. The two-level connection between the implant and gingival epithelium enables the adhesion of the gingival epithelium to the implant to be substantially improved. This improvement is not a mere mechanical fact, but also biological because of the greater quantity of connective tissue which can nest within said cavities, this greater quantity of vital tissue ensuring better antibody defence against biological attack.

The experimental data have shown that the diameter and length of the nanotubes strongly depend on the viscosity of the electrolyte used in the anodic oxidation and on the anodization time (J. M. Macak, Anodic growth of self-organized anodic TiO2 nanotubes in viscous electrolytes, Electrochimica Acta, 2006, vol. 52, Issue 3, pp. 1258-1264), hence enabling these two parameters to be graduated as required. Preferably the diameter of the nanotubes is between 10 and 300 nanometres and their length between 1 and 10 micrometres. The maximum width of the cavities is between 0.1 and 3.0 micrometres, while their length can even reach a few tens of micrometres. From tests carried out, it has been seen that the surface occupied by the cavities is conveniently 20-30% of the relative implant surface.

The cavity dimensions and the percentage of the area occupied by the cavities are controllable both during and after anodic oxidation by thermal treatment, as is known to experts of the art.

## Claims

1. A dental implant of titanium or its alloys, in which at least that surface of the implant intended to come into contact with the gingival epithelium has a structure of the titanium oxide nanotube type, with cavities of micrometric dimensions.

2. A dental implant as claimed in claim 1, wherein the diameter of the nanotubes is between 10 and 300 nanometres and their length between 1 and 10 micrometres.

3. A dental implant as claimed in claim 1, wherein the maximum width of the cavities is between 0.1 and 3.0 micrometres.

4. A dental implant as claimed in claim 1, wherein the surface occupied by the cavities is 20-30% of the relative implant surface.
